**Europäisches Patentamt**

**European Patent Office**

**Office européen des brevets**

(19)

(11) Veröffentlichungsnummer: **0 004 667**

**A1**

(12) # EUROPÄISCHE PATENTANMELDUNG

(21) Anmeldenummer: **79101034.1**

(22) Anmeldetag: **05.04.79**

(51) Int. Cl.²: **A 61 N 1/36**
**H 05 B 7/06**

(30) Priorität: **07.04.78 AT 2478/78**

(43) Veröffentlichungstag der Anmeldung:
**17.10.79 Patentblatt 79/21**

(84) Benannte Vertragsstaaten:
**CH DE FR GB IT NL SE**

(71) Anmelder: **Precimed S.A.**
**Chemin des Tourelles 17**
**CH-2400 Le Locle(CH)**

(72) Erfinder: **Mohl, Werner, Dr. Dr.**
**Lazarettgasse 14**
**Wien 9(AT)**

(74) Vertreter: **Buntz, Gerhard**
**Grenzacherstrasse 124 Postfach 601**
**CH-4002 Basel(CH)**

(54) **Kardialsonde.**

(57) Sonde zur Uebertragung elektrischer Signale zwischen einer im Patientenorgan angeordneten Elektrode und einer an anderer Stelle angebrachten Steuerungseinheit (Schrittmacher). Zur Anpassung an physiologische Unterschiede ist die Sondenlänge veränderbar. Dies wird dadurch erreicht, dass die schlauchförmige Sonde koaxial ineinandergestülpte Teilbereiche aufweist und die elektrischen Leiter vorzugsweise spiralig an bzw. in der Sondenwand verlaufen.

*FIG.1*

EP 0 004 667 A1

0004667

Precimed S.A., Le Locle (Schweiz)

PD 4701/102A

Kardialsonde

Die Erfindung betrifft eine intrakardiale Sonde, wie sie vor allem für künstliche Herzschrittmacher verwendet wird. Derartige bekannte intrakardiale Sonden weisen ein rohrförmiges Zuführleitungsteil, einen Sondenkopf mit wenigstens einer Elektrode und wenigstens einen elektrischen Leiter auf, über welchen die Elektrode mit einer Energiequelle verbindbar ist. Diese Sonden können hiebei eine oder zwei Elektroden aufweisen, wobei man im Falle der Verwendung einer unipolaren Elektrode üblicherweise die aktive Elektrode auf negatives Potential bringt und die zweite Elektrode, nämlich die indifferente Elektrode des Schrittmachers an einer beliebigen Stelle im menschlichen Körper anordnet. Bipolare Elektroden erfordern naturgemäss zwei elektrische Leitungen innerhalb der Zuführungsleitung. Derartige Sonden werden vor allem über

Bu/28.3.1979

ein Blutgefäss eingeführt und der Sondenkopf wird am Myocard befestigt. Bei der Einführung einer solchen Sonde über eine Vene wird der Sondenkopf endomyocardial angebracht. Prinzipiell ist aber auch eine epimyocardiale Anbringung der Sonde möglich, wobei dann die Zuleitung nicht über ein Blutgefäss erfolgen muss.

Es ist bereits bekannt, die Zuleitung in teleskopisch ineinanderschiebbare Abschnitte zu unterteilen, jedoch ist eine solche Ausführung mit dem Nachteil verbunden, dass die Zuleitung an den ineinandergeschobenen Stellen undicht wird. Die Einführung einer solchen Sonde erfolgt hiebei in an sich bekannter Weise unter Zuhilfenahme eines sogenannten Mandrins, durch welchen die mechanische Stabilität der Zuleitung während der Einführung wesentlich erhöht wird. Dieser Mandrin wird nach Anbringung der Sonde aus dem Zuleitungsteil wieder herausgezogen.

Die vorliegende Erfindung hat sich nun die Aufgabe gestellt, eine Zuleitung für eine solche Sonde zu schaffen, welche auch nach ihrer Anbringung noch Längenveränderungen zulässt. Die bekannten teleskopisch ineinandergeschobenen Abschnitte der Zuleitung erlauben lediglich ein Verlängern des freien Endes der Zuleitung. Eine Längerveränderung über die gesamte Länge der Sonde ist aber auf Grund der hohen Reibung zwischen den ineinandergeschobenen Teilbereichen der teleskopischen Abschnitte nicht möglich. Eine solche Längenveränderung der Zuleitung ist dann von besonderer Wichtigkeit, wenn die intrakardiale Sonde in Kleinkindern angebracht wird, welche naturgemäss durch Wachstum die Sondenzuleitung mechanisch beanspruchen. Eine Längenveränderung ist aber auch dann erforderlich, wenn bei Infektion des Ortes, an welchem die Energiequelle angeordnet wurde, die Energiequelle in andere subkutane Regionen verlegt werden muss. In diesem Fall muss in aller Regel der extravasale Teil der Sondenzuleitung verlängert werden. Es kann aber auch vorkommen, dass die Sonde im Zuge einer Herzoperation epimyocardial angesetzt wird. In diesem Fall verläuft die Sondenzuleitung durchwegs extravasal und ist im Gewebe relativ stärkeren Beanspruchungen ausgesetzt als der intravasale Teil der Sondenzuleitung bei einer endomyocardialen Anordnungsweise des Sondenkopfes.

Zur Lösung dieser Aufgabe eine mit einfachen Mitteln verlängerbare Sondenzuleitung zu schaffen, ist die vorliegende Erfindung im wesentlichen dadurch gekennzeichnet, dass der Zuleitungsteil von einem Schlauch gebildet ist, dessen Länge grösser ist als der Abstand zwischen dem Anbringungsort der Sonde und der Energiequelle, wobei die Wandungen über axiale Bereiche, welche kleiner als dieser Abstand sind, ineinander gestülpt sind und dass die elektrischen Leiter entlang der Innenwandung oder innerhalb der Wandung des Schlauches, vorzugsweise schraubenlinienförmig gewunden angeordnet sind. Dadurch, dass die Länge des Schlauches grösser ist als der Abstand zwischen Anbringungsort der Sonde und der Energiequelle, lässt sich der Ort der Energiequelle wahlweise operativ verlegen, ohne dass hiebei die Sondenzuleitung durch Anbringung weiterer Zuleitungsteile verlängert werden muss. Es genügt hiebei durch Zug an der Sondenzuleitung einzelne der axiale Bereiche, in welchen die Wandung des Schlauches ineinander gestülpt ist, zu strecken, wobei die Ausbildung der Sondenzuleitung als ineinander gestülpter Schlauch nach dem Ausziehen der Zuleitung auf eine grössere Länge elastische Rückstellkräfte im Sinne einer Verringerung der Länge der Sondenzuleitung vermeidet. Es ist für die erfindungsgemässe Ausbildung erforderlich, den elektrischen Leiter so innerhalb der Zuleitung anzuordnen, dass er bei einer Verlängerung der Sondenzuleitung nicht reisst. Vorzugsweise ist daher der elektrische Leiter schraubenlinienförmig gewunden angeordnet, da eine solche Ausbildung des elektrischen Leiters bei einer nachträglichen Längenveränderung des Zuleitungsschlauches den geringsten mechanischen Beanspruchungen unterworfen ist.

0004667

Die erfindungsgemässe Ausbildung ist vorzugsweise so getroffen, dass die Länge jedes axialen Bereiches des Schlauches, in welchem die Schlauchwandungen ineinander gestülpt sind, zumindest gleich dem Durchmesser des Schlauches ist und vorzugsweise dem 2- bis 10-fachen dieses Durchmessers entspricht. Diese Mindestbemessung der axialen Bereiche, in welchen die Schlauchwandungen ineinandergestülpt sind, hat zur Folge, dass elastische Kräfte, welche im Sinne einer Verlängerung des Schlauches wirksam werden könnten, weitgehend vermieden werden, da eine kürzere Bemessung des axialen Bereiches auf Grund der relativ starken Krümmung der Schlauchwandungen im Bereich der Faltung zur Streckung des Wandbereiches tendiert.

Um eine möglichst weitgehende Verlängerung der bereits verlegten Sondenzuleitung zu ermöglichen, ist die Ausbildung vorzugsweise so getroffen, dass die Summe der Längen der axialen Bereiche des Schlauches, in welchen die Schlauchwandungen ineinander gestülpt sind, zumindest gleich der Länge des Schlauches im ineinander gestülpten Zustand entspricht, wobei eine Verlängerung des Zuleitungsschlauches über das doppelte der ineinander geschobenen Länge dadurch ermöglicht wird, dass durch Faltung der Wandbereiche des Schlauches eine mehrfache, vorzugsweise 3- bis 5-fache Ueberlappung in zumindest einem Teil der axialen Bereiche, in welchen die Schlauchwandungen ineinander gestülpt sind, ausgebildet ist.

Ein wesentliches Kriterium, das an das Material des Zuleitungsschlauches gestellt wird, ist, dass es sich hiebei um ein elastisch aufweitbares Material handelt. Diese Anforderungen werden von den für Sondenzuleitungen bereits bekannten Materialien, insbesondere von Silikon-Kautschuk, erfüllt.

Die Erfindung wird nachfolgend an Hand von in der Zeichnung dargestellten Ausführungsbeispielen näher erläutert. In dieser zeigen Fig. 1 einen Teilbereich einer Sondenzuleitung, Fig. 2 einen weiteren Teilbereich einer solchen Sondenzuleitung und Fig. 3 einen Schnitt durch einen Teilbereich einer Wandung der Sondenzuleitung.

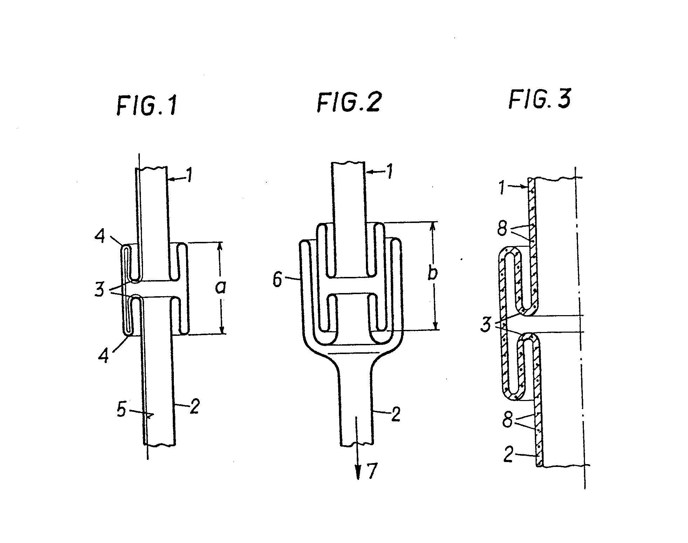

In Fig. 1 ist die Sondenzuleitung von einem Schlauch 1 gebildet. Die Wandungen 2 dieses Schlauches sind in einem axialen Bereich a ineinander gestülpt, wobei sich Faltstellen 3 und 4 der Wandungen 2 ergeben. Der elektrische Leiter ist mit 5 bezeichnet und verläuft längs der Wandung des 2 Schlauches 1. Aus Fig. 1 ist ersichtlich, dass die axiale Länge des Bereiches, in welchem die Wandungen 2 ineinander gestülpt sind, kleiner ist als der Abstand zwischen dem Anbringungsort der Sonde und der Energiequelle, da ja in Fig. 1 nur ein Teilbereich des Zuleitungsteiles dargestellt ist.

In Fig. 2 sind die Wandungen 2 des Schlauches 1 mehrfach übereinander gestülpt, wobei über einen Teil des axialen Bereiches b noch zusätzlich der Wandungsteil 6 an der Aussenseite überlappend angeordnet ist. Bei Zug am Ende des Schlauches 1 in Richtung des Pfeiles 7, d.i. in axialer Richtung des Schlauches, wird sich die Ueberlappung verringern und das für die Verlängerung der Sondenzuleitung erforderliche Material steht in ausreichendem Masse zur Verfügung.

In Fig. 3 ist in der Wandung 2 ein elektrischer Leiter 8 schraubenlinienförmig gewunden eingebettet. Bei dieser Ausbildung ist der elektrische Leiter 8 auch bei mehrfacher Uebereinanderstülpung von Teilbereichen der Wandung 2 nur geringen mechanischen Belastungen ausgesetzt und es besteht kaum die Gefahr eines Reissens der elektrischen Zuleitung bei der Verlängerung des Sondenzuleitung. Diese elektrischen Zuleitungen 8 können aber auch selbstverständlich in nicht dargestellter Weise an der Innenwand des Schlauches 1 anliegen, wodurch sich bei einer Verlängerung nur eine Vergrösserung der Ganghöhe der Schraubenwindung ergibt. Wenn die Windungen an der Innen-

0004667

wand des Schlauches anliegen, wird der Mandrin für die Einführung der Zuleitung nicht behindert und kann daher diese Zuleitungen nicht beschädigen.

0004667

## Patentansprüche

1. Sonde, insbesondere Kardialsonde mit einem rohrförmigen Zuleitungsteil, einem Sondenkopf mit wenigstens einer Elektrode und wenigstens einem elektrischen Leiter, über welchen die Elektrode mit einer Energiequelle verbindbar ist, dadurch gekennzeichnet, dass der Zuleitungsteil von einem Schlauch gebildet ist, dessen Länge grösser ist als der Abstand zwischen dem Anbringungsort der Sonde und der Energiequelle, wobei die Wandungen über axiale Bereiche, welche kleiner als dieser Abstand sind, ineinander gestülpt sind und dass die elektrischen Leiter entlang der Innenwandung oder innerhalb der Wandung des Schlauches, vorzugsweise schraubenlinienförmig gewunden angeordnet sind.

2. Sonde nach Anspruch 1, dadurch gekennzeichnet, dass die Länge jedes axialen Bereiches der Schlauches, in welchem die Schlauchwandungen ineinander gestülpt sind, zumindest gleich dem Durchmesser des Schlauches ist und vorzugsweise dem 2- bis 10-fachen dieses Durchmessers entspricht.

3. Sonde nach Anspruch 1 oder 2, dadurch gekennzeichnet, dass die Summe der Längen der axialen Bereiche des Schlauches, in welchen die Schlauchwandungen ineinander gestülpt sind, zumindest gleich der Länge des Schlauches im ineinander gestülpten Zustand entspricht.

4. Sonde nach einem der Ansprüche 1, 2 oder 3, dadurch gekennzeichnet, dass durch Faltung der Wandbereiche des Schlauches eine mehrfache, vorzugsweise 3- bis 5-fache Ueberlappung in zumindest einem Teil der axialen Bereiche, in welchen die Schlauchwandungen ineinander gestülpt sind, ausgebildet ist.

0004667

5. Sonde nach einem der Ansprüche 1 bis 4, dadurch gekennzeichnet, dass der Schlauch elastisch aufweitbar ist und insbesondere in an sich bekannter Weise aus Silikon-Kautschuk besteht.

\*\*\*

FIG.1

FIG.2

FIG.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 894 541 (EL-SHAFEI)<br>* Spalte 3, Zeile 4; Spalte 4, Zeilen 2-12; Figur 1 *<br>-- | 1,2,5 |
| | FR - A - 2 334 338 (DOW CORNING)<br>* Seite 3, Zeilen 16-31 *<br>-- | 1,3,4 |
| | FR - A - 2 301 760 (PRODUITS INDUSTRIELS DU D'OUEST)<br>* Anspruch 1 *<br>-- | 1,2 |
| | US - A - 3 683 932 (ADCOLE)<br>* Spalte 4, Zeilen 16-30 *<br>-- | 1,5 |
| | US - A - 3 466 742 (SINCLAIR)<br>* Spalte 2, Zeilen 4-7,42-55; Spalte 5, Zeilen 20-22 *<br>-- | 1,5 |
| | FR - A - 976 516 (ELOTANGE)<br>* Seite 2, linke Spalte, die letzten 7 Zeilen; rechte Spalte, Zeilen 1,2 *<br>-- | 1 |
| | US - A - 3 142 067 (LIEBIG)<br>* Spalte 1, Zeilen 8-14; Spalte 3, Zeilen 72-75 *<br>--<br>./. | 1 |

**KLASSIFIKATION DER ANMELDUNG (Int.Cl.²)**

A 61 N 1/36
H 05 B 7/06

**RECHERCHIERTE SACHGEBIETE (Int. Cl.²)**

A 61 N 1/36
H 05 B 7/06
A 61 M 25/00
A 61 F 1/24
F 16 L 51/02
A 61 M 1/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| Den Haag | 10-07-1979 | SIMON |

EPA form 1503.1  06.78

| | Europäisches<br>Patentamt | **EUROPÄISCHER RECHERCHENBERICHT** | 0004667<br>EP 79 10 1034 |
|---|---|---|---|

| | EINSCHLÄGIGE DOKUMENTE | | | KLASSIFIKATION DER<br>ANMELDUNG (Int.Cl.²) |
|---|---|---|---|---|
| Kategorie | Kennzeichnung des Dokuments mit Angabe, soweit erforderlich, der<br>maßgeblichen Teile | | betrifft<br>Anspruch | |
| | DE - A - 2 456 399 (KRONE)<br><br>* Anspruch 1; Figur 1 *<br><br>---- | | 1 | |
| | | | | RECHERCHIERTE<br>SACHGEBIETE (Int. Cl.²) |